(19) Europäisches Patentamt
European Patent Office
Office européen des brevets

(11) Publication number: **0 062 407 A1**

(12) # EUROPEAN PATENT APPLICATION

(21) Application number: **82301060.8**

(22) Date of filing: **02.03.82**

(51) Int. Cl.³: **C 07 D 498/04**, A 61 K 31/42 // (C07D498/04, 263/00, 205/00)

(30) Priority: **10.03.81 GB 8107504**

(43) Date of publication of application: **13.10.82 Bulletin 82/41**

(84) Designated Contracting States: **BE CH DE FR GB IT LI NL SE**

(71) Applicant: **BEECHAM GROUP PLC, Beecham House Great West Road, Brentford Middlesex (GB)**

(72) Inventor: **Gilpin, Martin Leonard, 42 Reeves Road, Reigate Surrey (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Therapeutic compounds containing beta-lactams.**

(57) A compound of formula (I):

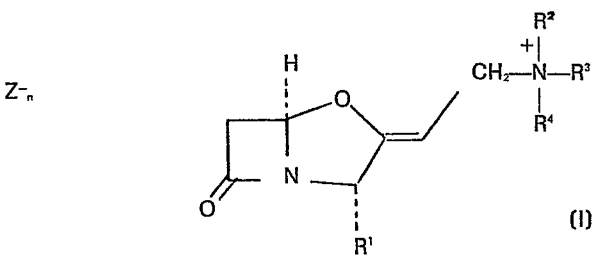

(I)

wherein R¹ is $CO_2^-$, $CO_2H$ or $CO_2R^5$ wherein $R^5$ is a group such that the grouping $CO_2R^5$ is a pharmaceutically acceptable in vivo hydrolysable ester;

$H^2$, $R^3$ and $R^4$ independently represent a $C_{1-12}$ hydrocarbon group optionally substituted with a functional substituent;

$Z^-$ is a negatively charged ion; and n is zero or one such that the compound of formula (I) is electronically neutral.

Their preparation and use is disclosed.

EP 0 062 407 A1

0062407

# THERAPEUTIC COMPOUNDS CONTAINING β-LACTAMS

This invention relates to a class of novel β-lactam compounds, to the process for their preparation and to pharmaceutical compositions containing them.

U.K. Patent Number 1566706 discloses *inter* alia the compounds of formula (A) and salts and esters thereof:

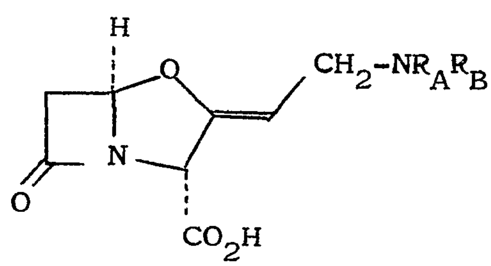

(A)

wherein $R_A$ is an inert organic group of up to 14 carbon atoms, and $R_B$ is an inert organic group of up to 16 carbon atoms, the $NR_AR_B$ group containing up to 22 carbon atoms. The compounds were disclosed to have antibacterial and β-lactamase inhibitory activity.

We have now found a novel class of compounds containing the quaternary ammonium group, which have

antibacterial and β-lactamase inhibitory activity.

Accordingly the present invention provides a compound of formula (I):

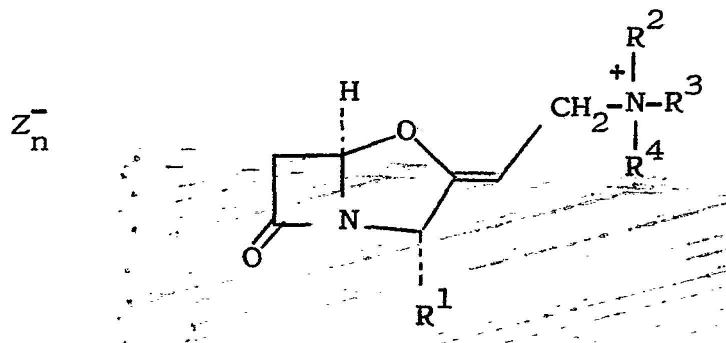

(I)

wherein $R^1$ is $CO_2^-$, $CO_2H$ or $CO_2R^5$ wherein $R^5$ is a group such that the grouping $CO_2R^5$ is a pharmaceutically acceptable <u>in vivo</u> hydrolysable ester;

$R^2$, $R^3$ and $R^4$ independently represent a $C_{1-12}$ hydrocarbon group optionally substituted with a functional substituent;

$Z^-$ is a negatively charged ion; and n is zero or one such that the compound of formula (I) is electronically neutral.

Examples of suitable pharmaceutically acceptable <u>in vivo</u> hydrolysable ester groups within $R^1$ include those which break down readily in the human body to leave the parent acid or its salts, for example acyloxyalkyl groups such as acetoxymethyl, pivaloyloxymethyl, α-acetoxyethyl and α-pivaloyloxyethyl groups; alkoxycarbonyloxyalkyl and α-ethoxycarbonyloxyethyl; dialkylaminoalkyl groups such as dimethylaminomethyl, dimethylaminoethyl, diethylaminomethyl or diethylaminoethyl; and lactone groups such as phthalidyl or dimethoxyphthalidyl.

Suitably $R^2$ to $R^4$ represent $C_{1-6}$ alkyl, aryl,benzyl, any of which may be substituted by up to three groups selected from halogen, $C_{1-6}$ alkoxy, $C_{2-6}$alkenyl, $C_{2-6}$ alkynyl, $C_{5-6}$ cycloalkyl, nitro, hydroxy, carboxy or $C_{1-6}$ alkoxy carbonyl.

Suitable groups $R^2$ to $R^4$ include $C_{1-3}$ alkyl, benzyl, nitrobenzyl and methoxybenzyl.

Preferably $R^2$ to $R^4$ are methyl, ethyl, benzyl, 4-nitrobenzyl and 4-methoxybenzyl.

Suitable $Z^-$ ions include halide, preferably $Z^-$ is chloride.

A preferred subgroup of compounds within formula (I) is the zwitterionic compounds represented by formula (II):

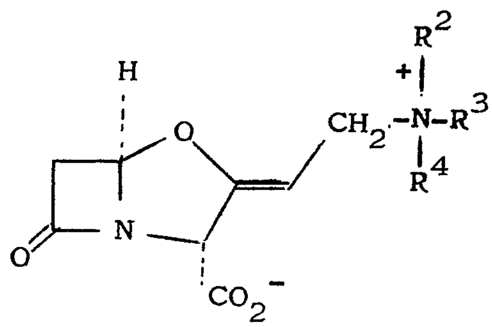

(II)

wherein $R^2$, $R^3$ and $R^4$ are as defined with respect to formula (I) hereinbefore.

The zwitteronic compounds of the formula (II) are normally and preferably in crystalline form. Compounds of this invention in crystalline form may be solvated, for example hydrated.

Particular compounds within formula (II) include
9(N-benzyl-N,N-dimethyl)ammonio-9-deoxyclavulanate,
9(N-benzyl-N-methyl-N-ethyl)ammonio-9-deoxyclavulanate,
9(N,N-dimethyl,N-ethyl)ammonio-9-deoxyclavulanate,
9(N,N,N-triethyl)ammonio-9-deoxyclavulanate, and
9(N,N,N-trimethyl)ammonio-9-deoxyclavulanate.

The present invention also provides a pharmaceutical composition which comprises a compound of this invention and a pharmaceutically acceptable carrier.

The compositions of the invention include those in a form adapted for oral, topical or parenteral use and may be used for the treatment of the infection in animals

especially mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water; sterile saline or the like and may also contain suspending agents such as polyvinylpyrrolidone, lecithin or the like. For use in such suspensions the zwitterionic compounds of this invention should be in the form of fine particles.

Injectable or infusable compositions of a compound of the invention are particularly suitable as high blood levels of the compound can occur after administration by injection of infusion. Thus, one preferred composition

aspect of this invention comprises a compound of the invention in sterile form and most suitably in sterile crystalline form.

Alternatively such compositions may be prepared in an acceptable oil suspending agent such as arachis oil or its equivalent. For use in such suspensions the compounds of this invention should be in the form of fine particles.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the formula may be present in the composition as sole therapeutic agent or it may be present together with other therapeutic agents such as, for example, as a penicillin or cephalosporin. Considerable advantages accrue from the inclusion of a penicillin or cephalosporin which shows instability to β-lactamases since the resulting composition shows enhanced effectiveness (synergy). Suitable penicillins cephalosporins or other β-lactam antibioticsfor inclusion in such synergistic compositions include not only those know to be highly susceptible to β-lactamases but also those which have a degree of intrinsic resistance to β-lactamases.

Suitable penicillins for inclusion in the compositions of this ivention include benzylpenicillin, phenoxymethyl-penicillin, carbenicillin, azidocillin, propicillin, ampicillin, amoxycillin, epicillin, ticarcillin, cyclacillin, pir-benicillin, azlocillin, mezlocillin, sulbenicillin, piperacillin, and other known penicillins including pro-drugs thereof such as their <u>in vivo</u> hydrolysable esters such as the acetoxymethyl, pivaloyloxymethyl, α-ethoxy-carbonyloxyethyl or phthalidyl esters of ampicillin, benzylpenicillin or amoxycillin, and aldehyde or ketone adducts of penicillins containing a 6-α-aminoacetamide side chain (such as hetacillin, metampicillin and analogous derivatives of amoxycillin) or α-esters of carbenicillin or ticarcillin such as their phenyl or indanyl α-esters.

Suitable cephalosporins for inclusion in the compositions of this invention include, for example, cefatrizine, cephaloridine, cephalothin, cefazolin, cephalexin, cephacetrile, cephamandole nafate, cephapirin, cephacetrile, cephamandole nafate, cephapirin, cephradine, 4-hydroxycephalexin, cefaparole, cephaloglycin, cefoperazone, and other known cephalosporins or pro-drugs thereof.

Such compounds are frequently used in the form of a salt or hydrate or the like.

Naturally if the penicillin or cephalosporin present in the composition is not suitable for oral administration then the composition will be adapted for parenteral administration,

Highly favoured penicillins for use in the compositions of this invention include ampicillin, amoxycillin, carbenicillin and ticarcillin. Such penicillins may be used as a pharmaceutically acceptable salt such as the sodium salt. Alternatively the ampicillin or amoxycillin may be used in the form of fine particles of the zwitterionic form (generally as ampicillin trihydrate or amoxycillin trihydrate) for use in an injectable suspension, for example, in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6 (wt/wt, based on pure free antibiotic equivalent).

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of *inter alia*, the respiratory tract, the urinary tract and soft tissues in humans and mastitis in cattle.

Normally between 50 the 3000 mg of the compounds of the invention will be administered each day of treatment but more usually between 100 and 1000 mg of the compounds of the invention will be adminstered per day, for example at 1-6 doses, more usually as 2, 3 or 4 doses. However for the treatment of more severe systemic infections or infections of particularly intransigent organisms higher doses may be used in accordance with

clinical practice.

The penicillin or cephalosporin in the synergistic composition of this invention will normally be present at approximately the amount at which it is conventionally used which will usually be expected to be from about 62.5 to 3000 mg per dose, more usually about 125, 250, 500 or 1000 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug thereof and from 25 to 500 mg of a compound of this invention.

Most suitably this form of composition will contain ampicillin trihydrate, ampicillin anhydrate, sodium ampicillin, hetacillin, pivampicillin hydrochloride, bacampicillin hydrochloride, or talampicillin hydrochloride. Most suitably this form of the composition will contain a zwitterionic compound of the formula (II) when in crystalline form.

Most suitably the preceding composition will contain from 200 to 700 mg of the penicillin component. Most suitably the preceding composition will comprise from 50 to 250 mg of a zwitterionic compound of the formula (II) preferably in crystalline form.

Such compositions may be adapted for oral or parenteral use except when containing an in vivo hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug thereof and from 50 to 500 mg of a compound of the invention.

Suitably this form of composition will contain di-sodium carbeniciilin. Suitably this form of the composition will contain di-sodium ticarcillin.

More suitably this form of the composition will contain from 75 to 250 mg of a zwitterionic compound of the formula (II) preferably in crystalline form. Such compositions containing di-salts of carbenicillin and ticarcillin will be adapted for parenteral administration.

The present invention also provides a method of treating bacterial infections in animals, in particular

humans or domestic mammals, which comprises the administration of a composition of this invention.

Commonly the infection treated will be due to a strain of Staphylococcus aureus, Klebsiella aerogenes, Escherichia coli, Proteus sp., Bacteroides fragilis or the like. The organisms believed to be most readily treated by an antibacterially effective amount of a compoound of this invention is Staphylococcus aureus. The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The present invention also provides a process for the preparation of a compound of formula (I) which process comprises reacting a compound of formula (III):

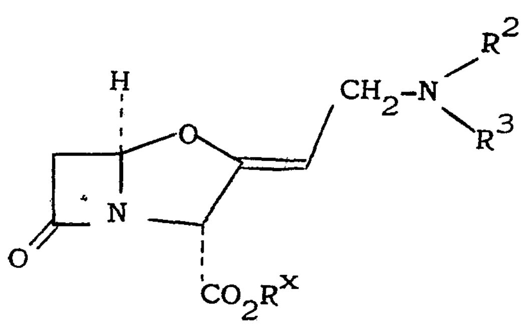

(III)

wherein $R^2$ and $R^3$ are as defined with respect to formula (I) hereinbefore and $R^x$ is a carboxy blocking group with a compound of formula $R^4$-X where X is a leaving group; and thereafter where necessary removing the carboxy blocking group $R^x$ and converting the compound to an in vivo hydrolysable ester.

Suitable groups X include halogen, such as iodine, bromine, or a sulphate group $-SO_4R^4$, an oxonium residue such as $-\overset{+}{O}(R^4)_2BF_4$ or an alkyl or aryl sulphonate such as methanesulphonate, or p-toluensulphonate. For example, where $R^4$ is methyl, suitable compounds $R^4$-X include methyl iodide, methyl bromide and dimethylsulphate.

The starting material of formula (III) is disclosed in U.K. Patent No. 1566706. The compounds of formula (III) may be prepared by reacting a secondary amine of formula (IV):

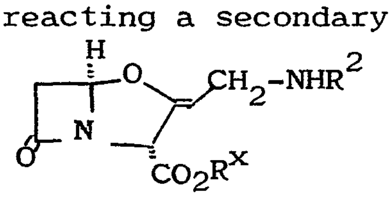

(IV)

with a compound $R^3$-X. For the preparation of compounds (I) where $R^3 = R^4$, compound IV may conveniently be reacted with two equivalents of compound $R^3$-X, without isolating the intermediate compound (III).

The compounds of formula (IV) may be prepared by reaction of the corresponding primary amine with a compound $R^2$-X. Similarly, when $R^2$=$R^3$=$R^4$, compounds (I) may be prepared by reacting the primary amine with 3 equivalents of compound $R^2$-X without isolating the intermediates (III) or (IV).

Suitable carboxyl-blocking derivatives for the group $-CO_2R^x$ in formula (III) include ester derivatives of the carboxylic acid. The derivative is preferably one which may readily be cleaved at a later stage of the reaction.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butylbenzyl 4-hydroxybenzyl, benzoylmethyl, p-nitrobenzyl, 4-pyridylmethyl, 2,2,2-trichloroethyl, 2,2,2-tribromoethyl, allyl, diphenylmethyl, triphenylmethyl, 2-benzyloxyphenyl, 4-methylthiophenyl, methoxymethyl, a silyl, stannyl or phosphorus-containing group, or an in vivo hydrolysable ester radical such as defined above.

The carboxylic group or a salt thereof may be regenerated from any of the above esters by usual methods appropriate to the particular $R^x$ group, for example, base-catalysed hydrolysis, or by enzymically-catalysed hydrolysis, or by hydrogention.

A preferred group $R^x$ is the silyl group, in particular the trimethylsilyl group which may be removed by aqueous hydrolysis.

The reaction is conveniently carried out in an inert organic solvent such as dimethylformamide, acetonitrile or methylene dichloride, preferably in the presence of a strong non-nucleophilic organic base, and at a non-extreme temperature for example $-10^{o}$ to $+50^{o}$, more usually $-5^{o}$ to $+20^{o}$ and conveniently in the range $-5^{o}$ to $+10^{o}$.

Suitable strong non-nucleophilic organic bases include 1,5-diazabicyclo[4.3.0.]non-5-ene (DBN).

The desired product may be obtained from the reaction mixtüre by evaporation of the solvent. Purification may be effected by crystallisation (for example before all the solvent has been evaporated off) or by column chromatography, for example using silica gel or cellulose and butanol/ethanol/water 4/1/1.

Certain compounds within formula (I) and protected derivatives thereof possess the additional utility as intermediates in the preparation of tertiary amines.

Accordinly the present invention also provides a process for the preparation of compounds of formula (V):

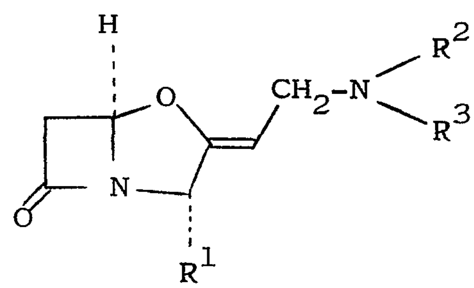

(V)

wherein $R^1$, $R^2$ and $R^3$ are defined with respect to formula (I);

which process comprises hydrogenation of a compound of formula (VI):

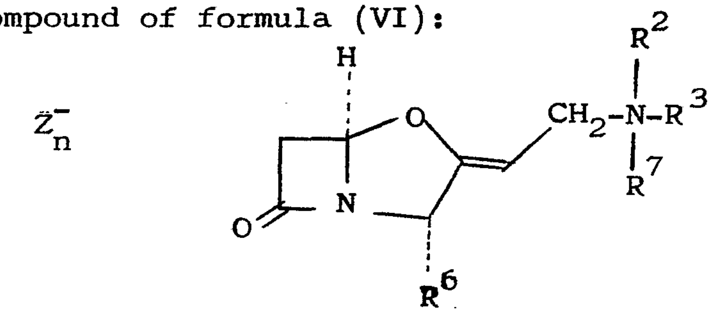

(VI)

wherein $R^6$ is $CO_2^-$, $CO_2H$ or $CO_2R^x$, and wherein $R^x$, $R^2$, $R^3$, n and Z are as defined above and $R^7$ is a group $R^4$ which is hydrogenolysable; and thereafter where necessary removing a carboxy blocking group $R^x$ and converting the product to a pharmaceutically acceptable <u>in vivo</u> hydrolysable ester.

Suitable groups $R^7$ include benzyl, benzyl substituted by up to three groups selected from halogen, $C_{1-6}$ alkyl, $C_{1-6}$ alkoxy, nitro, hydroxy, carboxy or $C_{1-6}$ alkoxy carbonyl.

The hydrogenation is normally carried out in the presence of a transition metal catalyst.

The catalyst we have preferred to use is palladium, for example in the form of palladium on carbon (charcoal), palladium on barium sulphate, palladium on calcium

carbonate or the like.

A favoured catalyst is palladium on carbon (sometimes referred to as palladium on charcoal); for example 5%, 10%, 20% or 30% palladium on carbon. The higher palladium content catalyst are particularly apt as smaller total weights of catalyst can be employed thereby avoiding possible problems associated with absorption of product onto the carbon.

A low, medium or high pressure of hydrogen may be used in this reaction, for example from 1 to 6 atmospheres. In general if the catalyst used contains a lower percentage of palladium (for example 5% or 10% palladium) then better yields of the desired product are obtained using a pressure of about 3 to 5 atmospheres of hydrogen, for example about 4 atmospheres of hydrogen. In general if the catalyst used contains a higher percentage of palladium (for example 20% or 30% palladium) then acceptable yields of the desired product may also be obtained at low and medium pressures of hydrogen, for example about 1 to 2 atmospheres of hydrogen. We have found it convenient to use an atmospheric or slightly superatmospheric pressure of hydrogen in conjunction with higher palladium content catalysts.

The reaction is normally carried out at a non-extreme temperature, for example from $0^{o}C$ to $30^{o}C$ and more usually from $12^{o}C$ to $25^{o}C$. It is generally convenient to carry out the reaction at ambient temperature.

Suitable solvents for carrying out the hydrogenation include ethanol, n-propanol, isopropanol, tetrahydrofuran, dioxane, ethyl acetate or mixtures of such solvents or such solvents in the presence of water. A favoured solvent is aqueous tetrahydrofuran. A further favoured solvent is a mixture of isopropanol, tetrahydrofuran and water.

The product may generally be isolated from the reaction mixture by filtering off the solids (the catalyst, which should be well washed to remove the product) and then evaporating the solvent, preferably under low pressure, to yield the initial product.

The desired product is normally obtained in crystalline form (unless it is an unsalted ester). Trituration under ethanol, isopropanol or the like $C_{1-4}$ alkanol or other conventional solvent such as a ketone, ether or ester solvent or other conventional solvent (for example of up to 6 carbon atoms and more suitably of up to 4 carbon atoms) may also be used to aid crystallisation.

The following examples illustrate the present invention.

Example 1

9-(N-Benzyl-N,N-dimethyl)ammonio-9-deoxyclavulanate

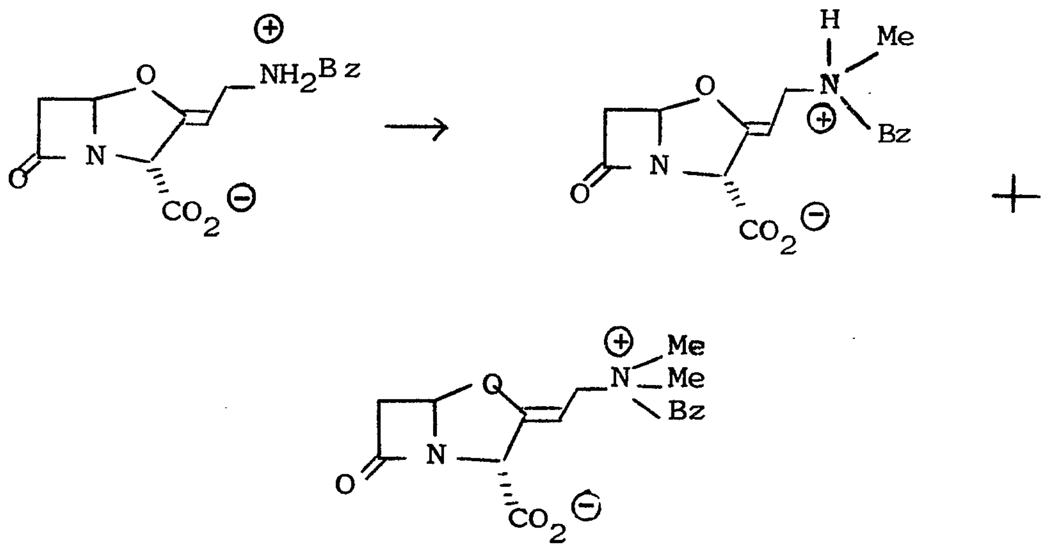

A suspension of the benzylamine (1.0 g, 3.5 mmol) in dry dimethylformamide (20 ml) was cooled (0°) and stirred during the addition of 1,5-diazabicyclo[4.3.0]non-5-ene (1.08 g, 8.7 mmol), and chlorotrimethylsilane (0.46 ml, 3.5 mmol). Stirring was maintained for a further 5 mins. Methyl iodide (0.33 ml, 5.2 mmol) was finally added and the reaction mixture stirred for 1.5 hrs, slowly allowing the temperature to reach 20°.

The dimethylformamide was evaporated and the residue chromatographed on silica gel. Elution with n-butanol/ethanol/water (4:1:1) gave recovered starting material (100 mg, 10%) and the monomethyl compound (527 mg, 50%) followed by the desired dimethylbenzylamine as a white solid (190 mg, 17%).

$\nu_{max}$ (KBr) 1790, 1685, 1620 $cm^{-1}$ $\delta$ ($D_2O$) 2.85 (6H, s, $C\underline{H}_3$), 3.05 (1H, d, $\underline{J}$ 17 Hz, β-lactam C$\underline{H}$H), 3.53 (1H, dd, $\underline{J}$ 17 and 3 Hz, β-lactam CH$\underline{H}$), 3.92 (2H, d, $\underline{J}$ 7 Hz, C(9)$\underline{H}$), 4.35 (2H, s, $NC\underline{H}_2Ph$), 4.91 (1H, t, $\underline{J}$ 7 Hz, vinyl $\underline{H}$), 5.05 (1H, s, C(3)$\underline{H}$), 5.76 (1H, d, $\underline{J}$ 3 Hz, β-lactam C$\underline{H}$), 7.45 (5H, s, Aryl $\underline{H}$) ppm.

Example 2

9-(N-Benzyl-N-ethyl-N-methyl)ammonio-9-deoxyclavulanate

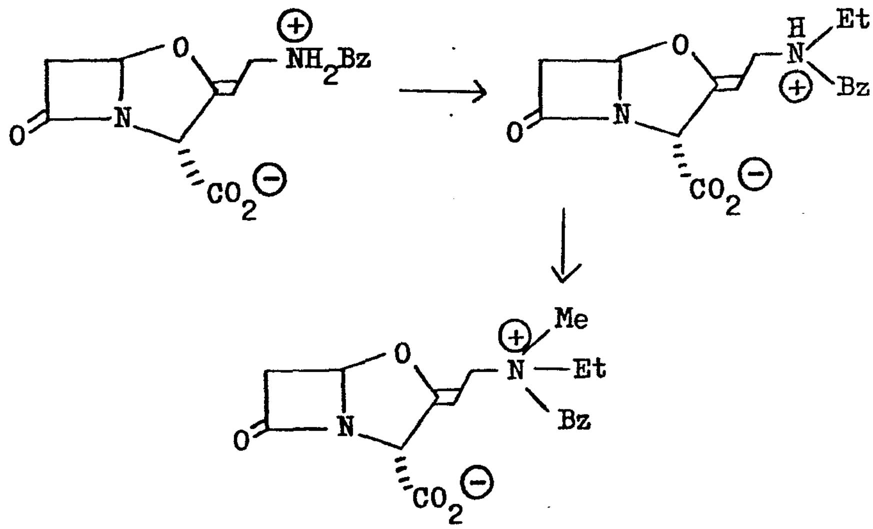

A suspension of the benzyl amine (1.0 g, 3.5 mmol) in dimethylformamide (20 ml) at $0^{o}$ was stirred and treated with 1,5-diazabicyclo[4.3.0.]non-5-ene (1.31 g, 10.5 mmol) followed by chlorotrimethylsilane (0.46 ml, 3.5 mmol). After 5 mins. ethyl iodid (0.56 ml, 1.09 g, 7.0 mmol) was added and the resulting solution stirred at $0^{o}$ for 0.5 hr and then at $20^{o}$ for a further 1 hr. Water (1 ml) was then added and the solvents evaporated. The residue was chromatographed on silica gel and elution with n-butanol/ethanol/water (4:1:1) gave recovered starting material (114 mg, 11%) followed by the monoethyl amine as a white solid (439 mg, 40%).

A suspension of the N-benzyl-N-ethyl amine (0.33 g, 1.04 mmol) in dry dimethylformamide (10 ml) was stirred at $0^{o}$ and treated with 1,5-diazabicyclo[4.3.0.]non-5-ene (130 mg, 1.04 mmol).

followed by chlorotrimethylsilane (0.14 ml, 1.04 mmol). After 5 mins methyl iodide (1.0 ml) was added and stirring at 0-20° maintained for a further 1.5 hrs. Water was then added and the solvents evaporated.

Chromatography on silica gel eluting with n-butanol/ethanol/water (4:1:1 → 1:1:1) afforded the desired product as a white solid (125 mg, 36%).

$\nu_{max}$ (KBr) 1790, 1700, 1680, 1620 $cm^{-1}$ δ ($D_2O$) 1.35 (3H, t, *J* 7Hz, $CH_2C\underline{H}_3$), 2.78 (3H, s, $NC\underline{H}_3$), 3.04 (1H, d, *J* Hz, β-lactam $C\underline{H}H$), 3.19 (2H, q, J 7 Hz, $NC\underline{H}_2CH_3$), 3.53 (1H, dd, *J* 17 and 3Hz, β-lactam $CH\underline{H}$), 3.87 (2H, d, *J* 7 Hz, C(9)$\underline{H}$), 4.34 (2H, s, $NC\underline{H}_2Ph$), 4.88 (1H, t, *J* 7 Hz, vinyl $\underline{H}$), 5.03 (1H, s, C(3)$\underline{H}$), 5.76 (1H, d, *J* 3 Hz, β-lactam $C\underline{H}$), 7.50 (5H, s, aryl $\underline{H}$), ppm.

Example 3

9-(N,N-dimethyl)amino-9-deoxyclavulanic acid

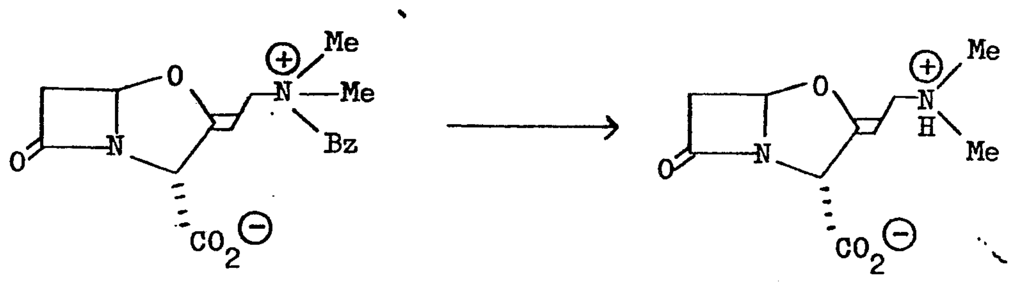

A solution of the 9-ammonio compound (130 mg, 0.41 mmol) in water (15 ml) was hydrogenated over 10% Pd/C (100 mg) for 3 hrs at ambient temperature and pressure.

The catalyst was removed by filtration through celite and the solvent removed under reduced pressure. After careful drying and trituration with ether/acetone the desired dimethyl amine was obtained as a pale solid (88 mg, 95%).

$\nu_{max}$ (KBr) 1790, 1680, 1620 $cm^{-1}$ $\delta(D_2O)$ 2.69 (6H, s, $NC\underline{H}_3$), 3.02 (1H, d, $\underline{J}$ 17 Hz, β-lactam (C$\underline{H}$H), 3.48 (1H, dd, $\underline{J}$ 17 and 3 Hz, β-lactam CH$\underline{H}$), 3.67 (2H, d, $\underline{J}$ 7 Hz, C(9)$\underline{H}$), 4.75 (1H, t, $\underline{J}$ 7 Hz, vinyl $\underline{H}$), 4.96 (1H, s, C(3)$\underline{H}$), 5.70 (1H, d, $\underline{J}$ 3 Hz, β-lactam C$\underline{H}$) ppm.

Example 4

9-(N-ethyl-N-methyl)amino-9-deoxyclavulanic acid

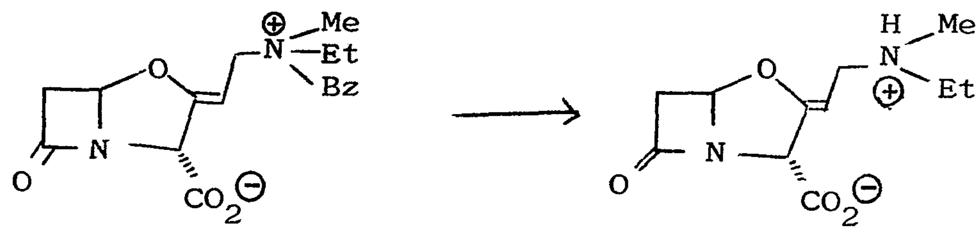

A solution of the 9-ammonio compound (122 mg) in water (20 ml) was hydrogenated over 10% Pd/C (60 mg) for 1-5 hrs. at ambient temperature and pressure.

The catalyst was filterred off and the solvent removed from the filtrate by evaporation under reduced pressure. The resulting oil was triturated with ether/acetone to afford the desired tertiary amine as a white solid (75 mg, 85%).

$\nu_{max}$ (KBr) 1780, 1680, 1630 $cm^{-1}$. $\delta$ ($D_2O$) 1.28 (3H, t, J 7 Hz, $CH_2C\underline{H}_3$), 2.72 (3H, s, $NC\underline{H}_3$), 3.08 (1H, d, J 17 Hz, β-lactam CH̲H), 3.10 (2H, q, J 7 Hz, $NC\underline{H}_2CH_3$), 3.55 (1H, dd, J 17 and 3 Hz, β-lactam CHH̲), 3.76 (2H, d, J 7 Hz, C(9)H̲), 4.79 (1H, t, J 7 Hz, vinyl H̲), 5.04 (1H, s, C(3)H̲), 5.80 (1H, d, J 3 Hz, β-lactam CH̲) ppm.

Example 5

9-(N,N-dimethyl-N-ethyl)ammonio-9-deoxyclavulanate

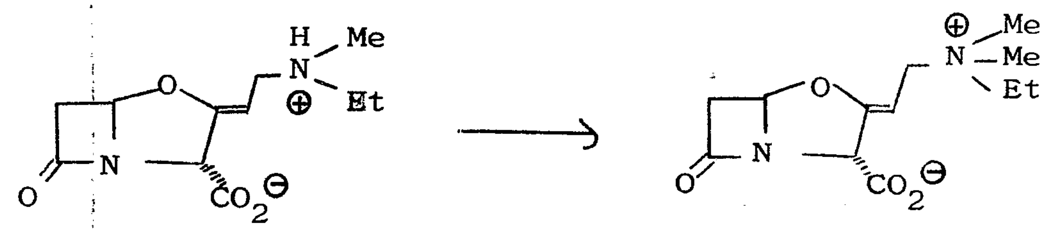

A solution of the tertiary amine (76 mg, 0.32 mmol) in dry dimethylformamide (10 ml) was cooled to 0° and stirred during the addition of 1,5-diazabicyclo[4.3.0.]non-5-ene (40 mg, 0.32 mmol) followed by chlorotrimethylsilane (0.04 ml, 0.32 mmol). After 5 mins. the reaction mixture was treated with methyl iodide (0.2 ml) and stirring at 0-20° continued for a further 2 hrs.

Water was then added to the reaction and the solvents evaporated under reduced pressure. Chromatography of the residue on silica gel, eluting with n-butanol/ethanol/water (1:1:1) gave recovered starting material (25 mg, 33%) followed by the desired ammonio compound as a white solid (15 mg, 19%).

$\nu_{max}$ (KBr) 1790, 1685, 1620 $cm^{-1}$. δ ($D_2O$) 1.34 (3H, t, J 7 Hz, $CH_2CH_3$), 2.96 (6H, s, $NCH_3$), 3.15 (1H, d, J 17 Hz, β-lactam CHH), 3.30 (2H, q, J 7 Hz, $NCH_2CH_3$), 3.60 (1H, dd, J 17 and 3 Hz, β-lactam CHH), 3.94 and 3.96 (2H, d, J 8 Hz, C(9)H), 4.88 (1H, t, J 8 Hz, vinyl H), 5.10 (1H, s, C(3)H), 5.82 (1H, d, J 3 Hz, β-lactam CH) ppm.

Example 6

9-N,N,N-triethylammonio-9-deoxyclavulanate

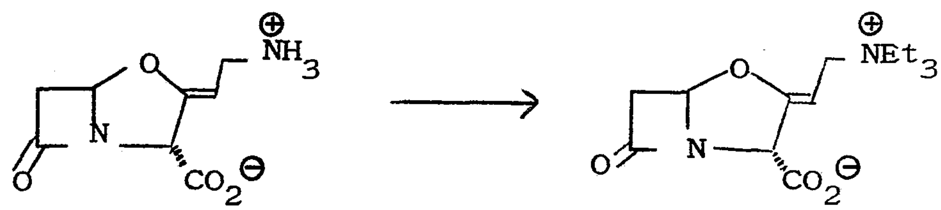

A suspension of the primary amine (200 mg, 1.0 mmol) in dry methylene dichloride (10 ml) was cooled ($0^o$) and stirred during the addition of 1,5-diazabicyclo [4.3.0.] non-5-ene (375 mg, 3.0 mmol) followed by chlorotrimethylsilane (0.13 ml, 1.0 mmol). After 5 mins at $0^o$ ethyl iodide (1 ml) was added and the reaction mixture stirred at $20^o$ for 2 hrs.

Water was added to the above mixture and the solvents evaporated. Chromatography of the residue on silica gel eluting with n-butanol/ethanol/water (4:1:1 → 1:1:1) gave the diethyl amine (29 mg, 11%) followed by the desired 9-ammonio derivative (37 mg, 13%).

$\nu_{max}$ (KBr) 1785, 1670, 1620 $cm^{-1}$. δ ($D_2O$) 1.30 (9H, t, J 7 Hz, $CH_2C\underline{H}_3$), 3.14 (1H, d, J 17 Hz, β-lactam C$\underline{H}$H), 3.20 (6H, q, J 7 Hz, $NC\underline{H}_2CH_3$), 3.61 (1H, dd, J 17 and 3 Hz, β-lactam CH$\underline{H}$), 3.89 (2H, overlapping d, J 7 Hz, C(9)$\underline{H}$), 4.85 (1H, t, J 7 Hz, vinyl $\underline{H}$), 5.09 (1H, s, C(3)$\underline{H}$), 5.81 (1H, d, J 3 Hz, β-lactam C$\underline{H}$) ppm.

Example 7

9-N,N,N,-trimethylammonio-9-deoxyclavulanate

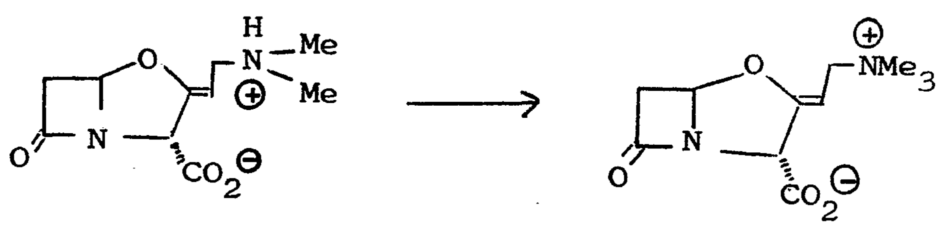

The dimethyl amine (116 mg, 0.515 mmol) in dry DMF (10 ml) was cooled ($0^o$) and stirred during the addition of 1,5-diazabicyclo[4.3.0.]non-5-ene (65 mg, 0.515 mmol) followed by chlorotrimethylsilane (0.07 ml, 0.515 mmol). After 5 mins. methyl iodide (0.5 ml) was added and the resulting solution was then stirred at $0^o$ for a further 1.5 hrs.

Water (0.5 ml) was added to the above reaction mixture and the solvents evaporated under reduced pressure. Chromatography on silica gel, eluting with n-butanol/ethanol/water (2:1:1 → 1:1:1), gave recovered starting material (20 mg, 17%) followed by the desired 9-ammonio (48 mg, 39%).

$\nu_{max}$ (KBr) 1785, 1685, 1620 $cm^{-1}$. δ ($D_2O$) 2.98 (9H, s, $NCH_3$), 3.09 (1H, d, J 17 Hz, β-lactam CHH), 3.56 (1H, dd, J 17 and 3 Hz, β-lactam CHH), 3.91 (2H, d, J 7 Hz, C(9)H), 4.88 (1H, t, J 7 Hz, vinyl H), 5.05 (1H, s, C(3)H), 5.79 (1H, d, J 3 Hz, β-lactam CH) ppm.

BIOLOGICAL DATA

Synergistic activity *in vitro* of some of the Compounds of the present invention.

| Compound | Inhibitor Conc | Staph. aureus Russell | Klebsiella aerogenes E70 | E. coli JT 39 |
|---|---|---|---|---|
| Amoxycillin alone | - | 1000 | 500 | ≥2000 |
| Amoxycillin with Compound of Example No 1 | 5 | 0.08 | 3.1 | 2 |
| | 1 | 0.62 | 6.2 | 8 |
| Amoxycillin with Compound of Example No 2 | 5 | - | 3.1 | 4 |
| | 1 | 0.16 | 12.5 | 16 |

Synergistic activity of quaternary amine derivatives with amoxycillin

| | MIC µg/ml amoxycillin | | |
|---|---|---|---|
| | S. aureus Russell | K. aerogenes E70 | E. coli JT39 |
| Amoxycillin alone | 500 | 1000 | 2000 |
| Amoxycillin 5.0µg/ml<br>with compound of Example 1.0µg/ml<br>No. 5 | 0.16<br>0.62 | 1.6<br>3.1 | 4.0<br>16.0 |
| Amoxycillin 5.0 µg/ml<br>with compound of 1.0 µg/ml<br>Example No. 6 | 0.08<br>0.62 | 3.1<br>6.2 | 8.0<br>16.0 |
| Amoxycillin 5.0 µg/ml<br>with compound of 1.0 µg/ml<br>Example No. 7 | 0.4<br>1.5 | 0.8<br>1.5 | 1.0<br>8.0 |

Antibacterial activity of quarternary amine derivatives

| Compound | S. aureus Russell | K. aerogenes E70 | E. coli JT39 |
|---|---|---|---|
| Example No. 5 | 31.0 | 250 | 62.0 |
| Example No. 6 | 31.0 | 500 | 125 |
| Example No. 7 | 4.0 | 62.5 | 16.0 |

MIC values in µg/ml.

CLAIMS :

1. A compound of formula (I):

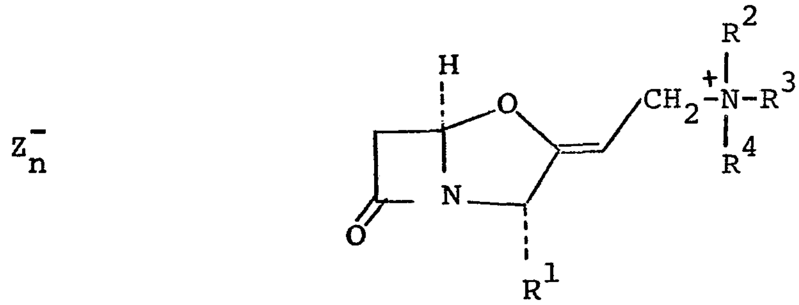

(I)

wherein $R^1$ is $CO_2$-, $CO_2H$ or $CO_2R^5$ wherein $R^5$ is a group such that the grouping $CO_2R^5$ is a pharmaceutically acceptable in vivo hydrolysable ester;

$R^2$, $R^3$ and $R^4$ independently represent a $C_{1-12}$ hydrocarbon group optionally substituted with a functional substituent;

$Z^-$ is a negatively charged ion; and n is zero or one such that the compound of formula (I) is electronically neutral.

2. A compound as claimed in claim 1 wherein $R^2$, $R^3$ and $R^4$ independently represent $C_{1-6}$ alkyl, aryl benzyl, any of which may be substituted by up to three groups selected from halogen, $C_{1-6}$ alkoxy, $C_{2-6}$ alkenyl, $C_{2-6}$ alkynyl, $C_{5-6}$ cycloalkyl, nitro, hydroxy, carboxy or $C_{1-6}$ alkoxycarbonyl.

3. A compound as claimed in claim 1 or claim 2 wherein $R^2$, $R^3$ and $R^4$ independently represent $C_{1-3}$ alkyl, benzyl, nitrobenzyl or methoxybenzyl.

4. A compound as claimed in any one of claims 1 to 3 wherein $R^2$, $R^3$ and $R^4$ independently represent methyl, ethyl, benzyl, 4-nitrobenzyl or 4-methoxybenzyl.

5. A compound as claimed in any one of claims 1 to 4 wherein $Z^-$ is chloride.

6. A compound as claimed in claim 1 having formula (II):

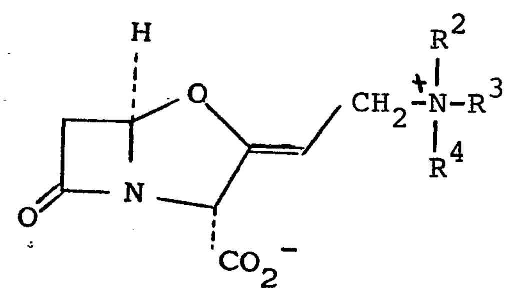

(II)

wherein $R^2$, $R^3$ and $R^4$ are as defined in claim 1.

7. A compound as claimed in claim 1 selected from the following:

9(N-benzyl-N,N-dimethyl)ammonio-9-deoxyclavulanate,
9(N-benzyl-N-methyl-N-ethyl)ammonio-9-deoxyclavulanate,
9(N,N-dimethyl-N-ethyl)ammonio-9-deoxyclavulanate,
9(N,N,N-triethyl)ammonio-9-deoxyclavulanate, and
9(N,N,N-trimethyl)ammonio-9-deoxyclavulanate.

8. A pharmaceutical composition comprising a compound as claimed in any one of claims 1 to 8 and a pharmaceutically acceptable carrier.

9. A process for the preparation of a compound as claimed in claim 1 which process comprises reacting a compound of formula (III):

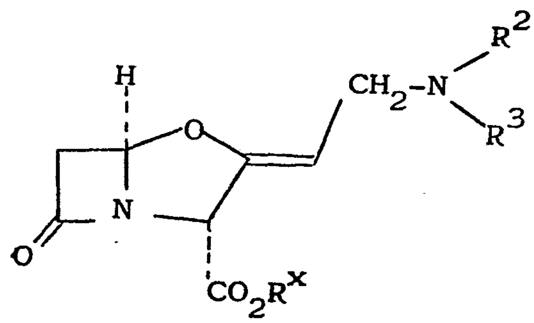

(III)

wherein $R^2$ and $R^3$ are as defined in claim 1 and $R^x$ is a carboxy blocking group with a compound of formula $R^4$-X where X is a leaving group; and thereafter where necessary removing the carboxy blocking group $R^x$ and converting the compound to an in vivo hydrolysable ester.

10. A process for the preparation of a compound of formula (V):

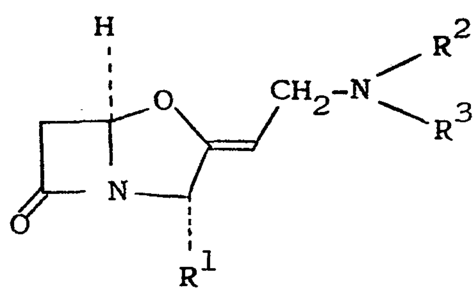

(V)

wherein $R^1$, $R^2$ and $R^3$ are defined in claim 1.

which process comprises hydrogenation of a compound of formula (VI):

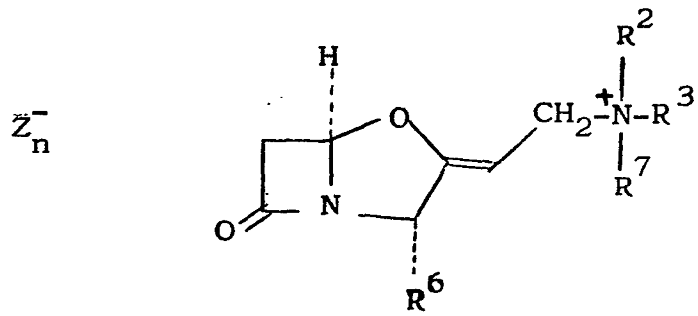

(VI)

wherein $R^6$ is $CO_2^-$, $CO_2H$ or $CO_2R^x$, and wherein $R^x$, $R^2$, $R^3$, n and Z are as defined in claim 9 and $R^7$ is a group $R^4$ which is hydrogenolysable; and thereafter where necessary removing a carboxy blocking group $R^x$ and converting the product to an *in vivo* hydrolysable ester.

European Patent Office

**EUROPEAN SEARCH REPORT**

0062407

Application number

EP 82 30 1060

**DOCUMENTS CONSIDERED TO BE RELEVANT**

| Category | Citation of document with indication, where appropriate, of relevant passages | Relevant to claim | CLASSIFICATION OF THE APPLICATION (Int. Cl. 3) |
|---|---|---|---|
| A | GB-A-1 570 988 (MERCK) *Pages 1-7* | 1,8-10 | C 07 D 498/04<br>A 61 K 31/42 //<br>(C 07 D 498/04<br>C 07 D 263/00<br>C 07 D 205/00 ) |
| A | DE-A-2 817 085 (BEECHAM) *Claims 1,70,95* | 1,8-10 | |
| D,A | GB-A-1 566 706 (BEECHAM) *Claims 1,45* | 1,8 | |
| | | | **TECHNICAL FIELDS SEARCHED (Int. Cl. 3)** |
| | | | C 07 D 498/00<br>A 61 K 31/00 |

The present search report has been drawn up for all claims

| Place of search | Date of completion of the search | Examiner |
|---|---|---|
| THE HAGUE | 03-05-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82